# EUROPÄISCHE PATENTSCHRIFT

(11) **EP 3 106 172 B1**
(45) Veröffentlichungstag und Bekanntmachung des Hinweises auf die Patenterteilung: **05.08.2020**
(21) Anmeldenummer: 16174594.8
(22) Anmeldetag: 15.06.2016
(51) Int. Cl.: A61K 31/717, A61K 31/14, A61K 31/08, A61K 9/00, A61K 45/06, A61L 2/18, A61P 17/02, A61P 31/02, A61P 31/04, A61K 38/00, A61K 38/02, A61K 38/03, A61L 2/00, A61K 9/06, A61K 9/08, A61K 47/10, A61K 47/42

(54) **ANTIMIKROBIELL AKTIVE MITTEL UND DEREN VERWENDUNG**
ANTIMICROBIAL AGENTS AND THEIR USE
MOYEN ACTIF ANTIMICROBIEN ET SON UTILISATION

(30) Priorität: 15.06.2015 DE 102015210913
(43) Veröffentlichungstag der Anmeldung: 21.12.2016
(73) Patentinhaber: B. Braun Melsungen AG, 34212 Melsungen (DE)
(72) Erfinder: Francois, Patrice, 74160 Saint-Julien-en-Genevois (FR); Landelle, Caroline, 73130 La Chambre (FR); Harbarth, Stephan, 1257 Landecy (CH); Arndt, Andreas, 6010 Kriens (CH); Henze, Heiko, 8052 Zürich (CH)
(74) Vertreter: dompatent von Kreisler Selting Werner - Partnerschaft von Patent- und Rechtsanwälten mbB

(56) Entgegenhaltungen:
- WO-A1-2014/091574
- WO-A1-2015/193677
- WO-A2-2006/018652
- DE-A1-102011 077 067
- JP-A- 2001 081 007
- US-A1- 2008 161 266
- MIJA AHN ET AL: "Poly-lysine peptidomimetics having potent antimicrobial activity without hemolytic activity", AMINO ACIDS., Bd. 46, Nr. 9, 25. Juni 2014 (2014-06-25), Seiten 2259-2269, XP055300520, AU ISSN: 0939-4451, DOI: 10.1007/s00726-014-1778-z
- DATABASE WPI Week 201262 Thomson Scientific, London, GB; AN 2012-L86743 XP002761609, & JP 2012 177901 A (TOMEI KK) 13. September 2012 (2012-09-13)
- PANDEY AJAY KUMAR ET AL: "Improved microbial biosynthesis strategies and multifarious applications of the natural biopolymer epsilon-poly-l-lysine", PROCESS BIOCHEMISTRY, Bd. 49, Nr. 3, 24. Dezember 2013 (2013-12-24), Seiten 496-505, XP028637809, ISSN: 1359-5113, DOI: 10.1016/J.PROCBIO.2013.12.009

## Beschreibung

Die vorliegende Erfindung betrifft Mittel zur Verwendung in der Behandlung und Prophylaxe von Infektionen durch multiresistente Bakterien sowie ein Mittel zur therapeutischen Verwendung in der Reduzierung multiresistenter Bakterien auf belebten Oberflächen gemäß den Ansprüchen. Darüber hinaus betrifft die vorliegende Erfindung ein Kit und einen Applikator, welche vorzugsweise in den erfindungsgemäßen Verfahren eingesetzt werden.

Die Haut und Schleimhaut des Menschen ist mit einer natürlichen bakteriellen Flora besiedelt. Überwiegend handelt es sich dabei um grampositive Mikroorganismen wie z. B. der nicht pathogene Staphylococcus epidermidis. Bei gesunden Menschen ist eine Besiedelung mit fakultativ pathogenen Keimen nicht von Relevanz, da die Haut einerseits eine Barrierefunktion erfüllt und zum Anderen das Immunsystem den Organismus vor einer Infektion schützt.

Anders stellt sich die Situation dar, wenn die Barrierefunktion von Haut und Schleimhaut beeinträchtigt ist und das Immunsystem aufgrund von Erkrankung seine ordnungsgemäße Funktion nicht mehr ausüben kann. In Krankenhäusern und Pflegeeinrichtungen treten zudem häufig pathogene Erreger auf, die gegen Antibiotika entweder teilweise oder vollständig resistent geworden sind. Gelangt ein Patient mit solchen Keimen in Kontakt, dann kann sich die Flora der Haut verschieben. Bei beeinträchtigter Barrierefunktion von Haut und Schleimhaut sind im Extremfall Infektionen möglich, die nur schwer zu behandeln sind. Ebenso kann es zu Infektionen kommen, wenn solche Erreger in Operationswunden gelangen. Die bekanntesten Erreger sind hierbei Methicillin-resistente Staphylokokken der Gattung Staphylococcus aureus (MRSA). Patienten mit MRSA-Besiedelung werden im Krankenhaus häufig durch ein geeignetes Screening erkannt. Kann eine MRSA-Besiedelung nachgewiesen werden und hat diese ein Risiko auf den Erfolg nachgeschalteter medizinischer Behandlung, wird meist versucht, den Patienten soweit zu sanieren, bis ein erneutes MRSA-Screening ein negatives Resultat ergibt.

Dazu wird die Haut kolonisierter Träger mit geeigneten antimikrobiell wirksamen Waschpräparaten, häufig in Kombination mit lokalen Antibiotika, behandelt. Die lokalen Antibiotika werden in der Regel zur Dekolonisation der Nasenschleimhaut - einem natürlichen Reservoir für Staphylokokken - eingesetzt. Besonders bewährt hat sich dabei Mupirozin, kommerziell beispielsweise erhältlich unter der Bezeichnung Baktroban und Turixin Nasensalbe (Glaxo Smith Kline). Eine der Grundlagen für die Sanierung von MRSA-Trägern wurde durch die Arbeit von Harbarth et al. (Antimicrob Agents Chemother. 1999, June; 43(6): 1412-6) gelegt, bei der die Wirksamkeit von Mupirozin allein und in Kombination mit einem Chlorhexidin-Waschpräparat verglichen wurde. Es ergab sich ein signifikanter Vorteil zugunsten der Kombination.

Über die Höhe von Sanierungsraten bei MRSA-Trägern gibt es in der Literatur verschiedene Angaben, in Abhängigkeit vom Studiendesign. Vergleicht man die Arbeiten, so stellt man fest, dass die Höhe der Sanierungsraten nicht mit der Qualität einzelner Präparate zusammenhängt, sondern durch weitere Faktoren, wie beispielsweise die allgemeine Umgebungshygiene und die Arbeit des Personals, beeinflusst wird. Beispielhaft sei die Arbeit von Krishna und Gibb (J. Hosp. Infect. 2010 Mar; 74(3): 199-203) erwähnt, die in einem Review zeigten, dass MRSA-Sanierungsraten mit Octenidin-basierten Präparaten - je nach Studie - zwischen 6 bis 75 % betrugen.

Ungeachtet dessen weist die Verwendung lokaler Antibiotika, wie Mupirozin, einen schwerwiegenden Nachteil auf. Bei Verwendung von Antibiotika mit spezifischem Wirkmechanismus auf Enzymsysteme besteht das Risiko der weiteren Resistenzentwicklung, so dass solche Präparate gegen die Bakterien mit der Zeit nur noch eingeschränkt oder überhaupt nicht mehr wirksam sind. Im Fall völliger Resistenz kann auch bei besonderer Umgebungs- und Personalhygiene keine erfolgreiche MRSA-Sanierung mehr erreicht werden.

Deshalb gibt es vermehrt Anstrengungen, bei der Sanierung der Nasenschleimhaut Präparate mit biozid wirksamen Substanzen zu verwenden. Bekannt sind hier z. B. Octenidindihydrochlorid und Polyhexanid.

DE 10 2012 215 511 A1 beschreibt ein Verfahren zur Herstellung halbfester Zubereitungen, die Bispyridinalkan enthalten und eine vergleichbare Wirksamkeit wie Mupirozin aufweisen sollen. Als Bispyridinalkan wird bevorzugt Octenidin eingesetzt.

DE 2005 045 145 A1 beschreibt halbfeste Zubereitungen enthaltend 0,005 bis 5 % Octenidindihydrochlorid, die unter Anderem zur Bekämpfung multiresistenter Erreger eingesetzt werden können. Zudem können die in der DE 2005 045 145 A1 beschriebenen Formulierungen Hydroxyethylcelluose enthalten.

Für die Sanierung der Haut haben sich neben Octenidindihydrochlorid und Polyhexanid vor Allem Chlorhexidingluconat, Triclosan, Benzalkoniumchlorid und Didecyldimethylammoniumchlorid etabliert. So beschreibt US 2010/0215626 A1 ein Kit zur Sanierung kolonisierter Träger, welches unter Anderem eine Waschlösung mit 4 % Chlorhexidingluconat, eine Mupirozinsalbe und eine Mischung zur bevorzugten Kultivierung von Staphylococcus epidermis auf der Haut enthält.

Biozide Substanzen und die daraus hergestellten Produkte weisen in der Regel ein nicht zu unterschätzendes cytotoxisches Potential auf, so dass Haut und Schleimhaut bei längerer Anwendung weiter geschädigt werden können. Speziell im Fall von Triclosan ist das Risiko der dermalen Resorption nicht unbeachtlich. Präparate auf Basis von Chlorhexidin enthalten als Verunreinigung N-Chloranilin, welches durch ein kanzerogenes Potential bekannt ist. Quaternäre Ammoniumverbindungen wie Benzalkoniumchlorid, Didecyldimethyl-ammoniumchlorid oder Benzethoniumchlorid können bereits in einer Konzentration von 0,1 % sensibilisierende Eigenschaften aufweisen, eine Verwendung auf geschädigter Haut oder gar bei bakteriell kolonisierten Wunden erscheint demnach nicht empfehlenswert.

Darüber hinaus ergibt sich, dass der Einsatz von Produkten, welche auf den zitierten traditionellen bioziden Aktivstoffen basieren, eine Reihe von nicht zu unterschätzenden Nachteilen aufweist. Ein alternativer Ansatz wurde in EP 2 255 821 A1 veröffentlicht, in dem ein Verfahren zur Herstellung pharmazeutisch wirksamer Produkte aus Biomassen lipidhaltiger Mikro- und Makroalgen, mariner Pilze, Cyanobakterien oder mariner Bakterien beansprucht wird, bei dem die Biomassen durch Homogenisierung oder Emulsionsbildung in lipidhaltige Mikro- und Nanopartikel umgewandelt werden. Die erfindungsgemäßen Mittel sollen das Wachstum von multiresistenten Staphylokokken hemmen. Die Standardisierung und Gewährleistung einer einheitlichen Wirksamkeit von Charge zu Charge ist dabei eine Herausforderung. WO 2015/193677 offenbart eine ophthalmologische Zusammensetzung enthaltend ε-Polylysin zur Behandlung von durch Methicillin-resistenten Staphylococus aureus (MRSA) verursachte Augeninfektionen.

Aus den genannten Gründen besteht ein Bedarf, geeignete Substanzen und Produkte mit Wirksamkeit gegen multiresistente Bakterien, die zur Sanierung von mit Bakterien kolonisierten Trägern und für die Wundbehandlung geeignet sind, zur Verfügung zu stellen.

Überraschend wurde nunmehr gefunden, dass Mittel, welche kationische Oligomere oder Polymere, insbesondere bestehend aus einer chemisch modifizierten Hydroxyalkylcellulose, enthalten, besonders geeignet sind zur Verwendung in der Behandlung und Prophylaxe von Infektionen durch multiresistente Bakterien, speziell von Infektionen durch multiresistente grampositive und/oder gramnegative Bakterien, insbesondere von Infektionen durch Betalaktam-Antibiotika oder Glycopeptid-Antibiotika resistente Bakterien. Es hat sich überraschend gezeigt, dass die erfindungsgemäßen Mittel nicht die Nachteile der herkömmlichen Biozide aufweisen. Es hat sich zudem überraschend gezeigt, dass die erfindungsgemäßen Mittel hervorragend zusammen mit weiteren bioziden Substanzen eingesetzt werden können, wobei der Anteil dieser zusätzlichen Biozide wirkungsvoll reduziert werden kann, um die zuvor geschilderten Nachteile zu kompensieren. Insbesondere hat sich gezeigt, dass eine synergistische Wirkungssteigerung über ein ausgeglichenes und breites Sprektrum an Bakterien erzielt werden kann, wenn zusätzlich biozide Komponenten, die ausgewählt sind aus der Gruppe der Polyaminosäuren, die ein oder mehrere freie -NH₂-Gruppen in dem Polymer aufweisen, zugegen sind.

Die erfindungsgemäßen Mittel können als flüssige oder halbfeste Zubereitung formuliert sein. Die erfindungsgemäßen Mittel können zusammen mit weiteren bioziden Komponenten formuliert werden und es hat sich überraschend gezeigt, dass nicht nur eine bakteriostatische Aktivität nachgewiesen wurde, sondern dass die erfindungsgemäßen Mittel in der Lage sind, die Keimbesiedelung von der Haut- und Schleimhautoberfläche des Patienten unter Praxisbedingungen zu reduzieren und dabei vor allem Antibiotika-resistente Bakterien über einen längeren Zeitraum von bis zu 28 Tagen dauerhaft zu eradizieren.

Gegenstände, die nicht durch den Schutzumfang der Ansprüche umfasst sind, sind nicht Teil der beanspruchten Erfindung. Gegenstand der vorliegenden Erfindung ist ein Mittel zur Verwendung in der therapeutischen Behandlung oder Prophylaxe von Infektionen durch Betalaktam-Antibiotika oder Glycopeptid-Antibiotika resistente Bakterien, umfassend
a) 0,05 bis 4 Gew.-% eines kationischen Oligomers oder Polymers, bestehend aus einer chemisch modifizierten Hydroxyalkylcellulose, und
b) 0,001 bis 2 Gew.-% Polylysin und/oder Polyarginin,
c) 0,1 bis 0,8 Gew.-% nicht-ionisches und/oder amphoteres Tensid, und
d) mindestens 85 Gew.-% Wasser und/oder Polyalkylglykole, wobei die Gewichtsangaben jeweils bezogen sind auf das Gesamtgewicht des Mittels.

Diese Mittel sind insbesondere geeignet zur Verwendung in der Behandlung oder Prophylaxe von Infektionen durch multiresistente Bakterien, speziell multiresistente grampositive und/oder gramnegative Bakterien, insbesondere durch Betalaktam-Antibiotika oder Glycopeptid-Antibiotika-resistente Bakterien.

Als multiresistente Bakterien werden solche Bakterienstämme bezeichnet, die mindestens gegen zwei Antibiotika, insbesondere Betalaktam-Antibiotika und/oder Glycopeptid-Antibiotika, resistent sind.

In einer bevorzugten Ausführungsform sind die Bakterienstämme gegen Methicillin und/oder Oxacillin und/oder Carbapenem und/oder Vancomycinresistent.

In einer Ausführungsform sind die Bakterienstämme resistent gegen ein oder mehrere Antibiotika ausgewählt aus der Gruppe bestehend aus Methicillin, Oxacillin, Carbapenem und Vancomycin.

In einer weiter bevorzugten Ausführungsform sind die Bakterien Methicillin resistente Staphylokokken oder Oxacillin resistente Staphylokokken oder Carbapenem resistente Pseudomonaden oder Vancomycin resistente Enterokokken, insbesondere Methicillin resistente Staphylokokken der Gattung Staphylococcus aureus (MRSA) oder Oxacillin resistente Staphylokokken der Gattung Staphylococcus aureus (ORSA) oder Carbapenem resistente Pseudomonaden der Gattung Pseudomonas aeruginosa oder Vancomycin resistente Enterokokken der Gattung Enterococcus faecium (VRE).

Obwohl nicht speziell erwähnt, ist davon auszugehen, dass Ausführungsformen der Mittel auch gegen weitere antibiotikaresistente Bakterien und Pilze wirksam sind sowie Sporenbildner wie z. B. Clostridium difficile am Auskeimen hindern.

Die erfindungsgemäßen Mittel werden bevorzugt topisch, insbesondere auf Haut oder Schleimhaut oder Wunden appliziert. In einem Aspekt der vorliegenden Erfindung werden die erfindungsgemäßen Mittel auf die Nasenschleimhaut appliziert. Dies kann beispielsweise durch Sprühen des Mittels, wie etwa mit Hilfe eines Nasensprayapplikators erfolgen oder durch Auftragen des Mittels in Form einer Salbe oder Creme.

### Komponente a)

Die kationischen Oligomere oder Polymere basieren bevorzugt auf Hydroxyethylcellullosen und/oder Hydroxyalkylstärken, die durch Umsetzung chemisch derart modifiziert werden können, dass sie kationische Ladungen tragen. Bevorzugt weisen die kationischen Oligomere oder Polymere quaternäre Ammoniumgruppen auf. Diese können beispielsweise durch dem Fachmann bekannte Umsetzungen von Epoxygruppen-tragenden Ammoniumverbindungen mit den Hydroxyalkylstärken erfolgen. In einer bevorzugten Ausgestaltung ist das kationische Oligomer oder Polymer eine chemisch modifizierte Hydroxyethylcellulose. Alternativ können auch Hydroxpropylcellulosen chemisch modifiziert werden.

Die chemische Derivatisierung der Hydroxyalkylcellulosen, insbesondere der Hydroxyethylcellulosen, kann in einer bevorzugten Ausgestaltung zur Ausbildung von Kammpolymeren führen, welche eine Hauptkette und mehrere Seitenketten aufweisen. Bevorzugt weisen das kationische Oligomer oder Polymer kationische Ladungen an den Seitenketten, insbesondere quaternäre Ammoniumgruppen in den Seitenketten auf.

Es wurde überraschend gefunden, dass die Wirksamkeit der kationischen Oligomere oder Polymere mit zunehmender Ladungsdichte zunimmt.

In einer Ausgestaltung der vorliegenden Erfindung weist das kationische Oligomer oder Polymer eine Ladungsdichte von wenigstens 0,1 meq/g, bevorzugt wenigstens 1.0 meq/g, weiter bevorzugt wenigstens 2.0 meq/g, besonders bevorzugt wenigstens 3.0 meq/g, speziell bevorzugt 4.0 meq/g und insbesondere wenigstens 5.0 meq/g oder wenigstens 6.0 meq/g, bestimmt gemäß Kolloidtitration, auf.

Weiterhin bevorzugt ist das kationische Oligomer oder Polymer ein Hydroxyethylcellulose umfassendes Copolymer.

Bevorzugt sind die chemisch modifizierten kationischen Hydroxyalkylcellulosen, die unter Verwendung der INCI Nomenklatur als Polyquaternium-4, Polyquaternium-10 und Polyquaternium-24 bezeichnet werden.

In einem Aspekt der Erfindung ist das kationische Oligomer oder Polymer ausgewählt aus der Gruppe bestehend aus Polyquaternium-4, Polyquaternium-10 und Polyquaternium-24.

Besonders bevorzugt sind dabei Diallyldimethylammonium chloridhydroxyethylcellulose Copolymer (Polyquaternium-4), Hydroxyethylcellulose, die mit Trimethylammonium substituierten Epoxiden umgesetzt wird (Polyquaternium-10) und Polyquaternium-4/Hydroxypropyl Starch Copolymer. Polyquaternium-4 ist kommerziell erhältlich, z. B. Celquat L-200 Polymer oder Celquat H-100 Polymer des Herstellers Akzo Nobel. Ebenso ist Polyquaternium 10 kommerziell erhältlich, z. B. Celquat SC-230M Polymer und CELQUAT SC-240C polymer des Herstellers Akzo Nobel. Alternativ sind die Polymere unter dem Namen Ucare von Dow erhältlich. Die einzelnen Typen Polyquaternium-4 und Polyquaternium-10 unterscheiden sich hinsichtlich der Viskosität. Polyquaternium-4/Hydroxypropyl Starch Copolymer ist von Akzo Nobel erhältlich unter der Bezeichnung Celquat LS-50 Polymer.

Polyquaternium-24 ist eine Hydroxyethylcellulose, die mit Lauryldimethylammonium substituierten Epoxiden hergestellt wird.

Besonders gute Ergebnisse hinsichtlich Wirksamkeit und gleichzeitiger Haut- beziehungsweise Schleimhautverträglichkeit konnte mit Polyquaternium-4 erzielt werden.

In einer weiteren Ausführungsform der vorliegenden Erfindung enthalten die Mittel Mischungen unterschiedlicher kationischer Oligomere oder Polymere, beispielsweise 2 oder mehrere kationische Oligomere oder Polymer bestehend aus chemisch modifizierten Hydroxyalkylcellulosen. Bevorzugt sind Mischungen aus Polyquaternium-4 und Polyquaternium-10.

Es hat sich überraschend gezeigt, dass der Einsatz der kationischen Oligomere oder Polymere bestehend aus einer chemisch modifizierten Hydroxyalkylcellulose selbst in geringen Konzentrationen eine hohe Wirksamkeit aufweist.

In einer weiteren Ausgestaltung der vorliegenden Erfindung enthält das Mittel das kationische Oligomer oder Polymer in einer Menge bevorzugt von 0,1 bis 3 Gew.-%, speziell von 0,15 bis 2 Gew.-%, insbesondere von 0,25 bis 1 Gew.-%, jeweils bezogen auf das Gesamtgewicht des Mittels.

Das kationische Oligomer oder Polymer ist bevorzugt antimikrobiell und liegt vorzugsweise in einer antimikrobiell wirksamen Menge vor.

### Komponente b) (biozide Komponente)

Ein weiterer Bestandteil des erfindungsgemäßen Mittels ist die Komponente b), die in einer Menge von 0,001 bis 2 Gew.-%, bezogen auf das Gesamtgewicht des Mittels, vorliegt.

Komponente b) ist Polylysin und/oder Polyarginin. Die Komponente b) ist von der Komponente a) unterschiedlich.

Durch den Einsatz der kationischen Oligomere oder Polymere (Komponente a)), bestehend aus einer chemisch modifizierten Hydroxyalkylcellulose, kann die Menge der bioziden Komponente (Komponente b)) erheblich reduziert werden. Die Biozide (Komponente b)), die sich von der Komponente a) unterscheiden, liegen daher in einer Menge von zwischen 0,001 und 2 Gew.-%, insbesondere von 0,01 bis 1 Gew.-% und speziell von 0,05 bis 0,5 Gew.-%, jeweils bezogen auf das Gesamtgewicht des Mittels, vor.

Sofern die erfindungsgemäßen Mittel eine oder mehrere biozide Komponenten enthalten, sind sie so ausgestaltet, dass eine sehr gute Haut und Schleimhautverträglichkeit resultiert. Die zentrale Funktion der bioziden Komponenten besteht in der Konservierung des erfindungsgemäßen Produktes gegen mikrobiellen Verderb durch Bakterien und insbesondere Pilze, aber auch in einer synergistischen Wirkungssteigerung und Ausgeglichenheit der Wirkung gegen verschiedenste Bakterienstämme.

Die Komponente b) ist Polylysin und/oder Polyarginin.

Die Komponente b) liegt bevorzugt als Homopolymer vor, dass heisst es liegen ausschließlich Aminosäuremonomereinheiten einer einzelnen Aminosäure, nämlich Lysin oder Arginin vor.

Es hat sich als vorteilhaft herausgestellt, die Monomereinheiten der Polyaminosäuren auf definierte Bereiche zu begrenzen. In einer bevorzugten Ausführungsform ist die Komponente b) eine Polyaminosäure, die 15 bis 50, vorzugsweise 20 bis 40 und speziell 25 bis 35 Aminosäure-Monomereinheiten, Lysin und/oder Arginin, aufweist.

Das mittlere Molekulargewicht der Polyaminosäure liegt bevorzugt zwischen 1500 bis 6500, weiter bevorzugt von 2200 bis 6000 und insbesondere von 3200 bis 5500 g/mol.

In einer weiteren Ausführungsform weisen die Mittel Polylysin, speziell epsilon-Polylysin auf. Es hat sich überraschend gezeigt, dass insbesondere die Kombination von Polylysin mit der Komponente a) in den erfindungsgemäßen Mitteln zu einer Wirkungssteigerung führt, aber gleichzeitig eine hervorragende Hautverträglichkeit, insbesondere der Schleimhäute, bietet.

Prinzipiell können sowohl das alpha- als auch das epsilon-Polylysin in der D- oder L-Form zu Einsatz kommen.

Es hat sich jedoch gezeigt, dass aus der Gruppe der Polylysine insbesondere das epsilon-Polylysin, speziell das epsilon-Poly-L-lysin, hervorragende Wirkeigenschaften zeigt. Polylysine sind kommerziell erhältlich und werden von verschiedenen Herstellern, z.B. als 25 % Lösung des Anbieters DKSH oder der JNC Corporation angeboten.

In einer Ausführungsform der vorliegenden Erfindung können die Mittel weitere biozide Komponenten enthalten, die von Komponente a) und Komponente b) verschieden sind und beispielsweise ausgewählt sind aus der Gruppe bestehend aus Alkoholen, organischen Säuren, quaternären Ammoniumverbindungen, Biguaniden, Octenidin Salzen und Iodopropinylbutylcarbamat.

In einer Ausführungsform enthalten die Mittel Alkohole, bevorzugt Monoalkohole, weiter bevorzugt aliphatische Monoalkohole, die gegebenenfalls mit aromatischen Resten substituiert sein können und insbesondere Monoalkohole, die 2 bis 10 oder 3 bis 9 Kohlenstoffatome aufweisen. In einer bevorzugten Ausführungsform sind die Alkohole, die als zusätzliche Biozide zum Einsatz kommen können, ausgewählt aus der Gruppe bestehend aus Ethanol, Propan-1-ol, Propan-2-ol, Phenoxyethanol, Phenylethanol, Phenoxypropanol und Benzylalkohol.

Der Einsatz von Alkoholen ist besonders bevorzugt, da diese zusätzlich als Lösevermittler dienen können.

Bei den quaternären Ammoniumverbindungen sind besonders solche bevorzugt, die nur eine quaternäre Ammoniumgruppe aufweisen. In einer Ausführungsform enthalten die erfindungsgemäßen Mittel quaternäre Ammoniumverbindungen ausgewählt aus der Gruppe bestehend aus Benzethoniumchlorid, Didecyldimethylammoniumchlorid, Benzyldimethyl-ammoniumchlorid, Alkyldimethylethylbenzylammoniumchlorid, Cetrimid und Mecetroniumetilsulfat. Die Menge und Auswahl der quaternären Ammoniumverbindungen wird so gewählt, dass sie die Verträglichkeit auf Haut, Schleimhaut und Wunden nicht negativ beeinflussen.

Bei den Biguaniden handelt es sich insbesondere um die Salze des Chlorhexidins, wie beispielsweise Chlorhexidindigluconat, Chlorhexidindiacetat, Chlorhexidindihydrochlorid oder um die Salze des Polyhexamethylenbiguanids, wie etwa Polyhexamethylenbiguanid Hydrochlorid (z. B. kommerziell erhältlich als Cosmocil PG ex Lonza AG).

Als Octenidin Salz hat sich beispielsweise das Octenidindihydrochlorid als besonders geeignet herausgestellt.

Die organischen Säuren sind insbesondere ausgewählt aus der Gruppe bestehend aus Benzoesäure, Sorbinsäure, Dehydracetsäure, 4-Hydroxybenzoesäure. Es können jedoch auch deren Ester zum Einsatz kommen, wie beispielsweise der 4-Hydroxybenzoesäurealkylester.

In einer speziellen Ausführungsform der vorliegenden Erfindung enthalten die Mittel Polyquaternium-4 und/oder Polyquaternium-10 in Kombination mit einem Biozid gemäß Komponente b).

Besonders gute Ergebnisse können mit der Kombination aus Polyquaternium-4 und epsilon-Polylysin erzielt werden.

In einer bevorzugten Ausführungsform beträgt das Gewichtsverhältnis von Komponente a) zu Komponente b) 100:1 bis 1:10, weiter bevorzugt 50:1 bis 1:5, insbesondere 25:1 bis 1:1 und speziell 20:1 bis 2:1.

Die Mittel liegen bevorzugt in Form einer Lösung, einer Emulsion, eines Gels, einer Salbe oder Creme vor.

Die erfindungsgemäßen Mittel können je nach galenischer Formulierung weitere Inhaltsstoffe aufweisen. Die weiteren Inhaltsstoffe können in Mengen bis zu 99,999 Gew.-% in den Mitteln vorliegen. Typischerweise können diese Zusatzstoffe in Mengen zwischen etwa 80 und 99.9 Gew.-%, insbesondere zwischen etwa 85 und 99.7 Gew.-%, speziell zwischen 95 und 99.6 Gew.-%, jeweils bezogen auf das Gesamtgewicht des Mittels, vorliegen.

Die erfindungsgemäßen Mittel liegen bevorzugt als wässerige Mittel vor. Die Mittel sind typischerweise bei Raumtemperatur (20 °C) flüssig oder halbfest bis pastös. Je nach Einsatzgebiet können die Mittel einen hohen Anteil an Wasser aufweisen. In einer Ausführungsform weisen die erfindungsgemäßen Mittel Wasser in einer Menge von mindestens 85 Gew.-%, weiter bevorzugt oberhalb von 87 Gew.-%, besonders bevorzugt oberhalb von 89 Gew.-%, insbesondere oberhalb von 95 Gew.-% und speziell oberhalb von 98 Gew.-% oder oberhalb 99 Gew.-%, auf, wobei die Gewichtsangaben bezogen sind auf das Gesamtgewicht des Mittels.

In einem weiteren Aspekt der Offenbarung enthalten die Mittel Wasser in einer Menge von weniger als 25 Gew.-%, vorzugsweise weniger als 20 Gew.-%, insbesondere weniger als 15 Gew.-%, beispielsweise zwischen 5 und 12 Gew.-%, jeweils bezogen auf das Gesamtgewicht des Mittels. Mittel mit einem geringeren Wassergehalt können beispielsweise zur Herstellung von Salben, beispielsweise zur Behandlung der Nasenschleimhaut verwendet werden.

Die weiteren Bestandteile der erfindungsgemäßen Mittel können zur Ausbildung einer mikrobiologisch inaktiven Hilfsstoffmatrix verwendet werden, beispielsweise für eine wässrige Lösung, eine Gelgrundlage, eine hydrophile oder eine hydrophobe Salbengrundlage oder auch für die Einbettung in eine Öl in Wasser Emulsion oder in eine Wasser in Öl Emulsion.

Sofern nicht explizit anders angegeben beziehen sich die Gewichtsangaben in Gewichtsprozent (Gew.-%), jeweils bezogen auf das Gesamtgewicht des Mittels.

Die Mittel enthalten als weitere Bestandteile nichtionische und/oder amphotere Tenside, in einer Menge von 0,1 bis 0,8 Gew.-%.

Es hat sich gezeigt, dass die Anwesenheit von nichtionischen und/oder amphoteren Tensiden die antimikrobielle Wirksamkeit der erfindungsgemäßen Mittel positiv beeinflussen kann. Die erfindungsgemäßen Mittel enthalten daher zusätzlich ein nichtionisches und/oder ein amphoteres Tensid.

Als nichtionische Tenside eignen sich vorzugsweise Fettalkoholethoxylate, insbesondere C₈₋₂₂-Fettalkohole mit 5 bis 30 Ethoxylateinheiten, speziell C₁₀₋₁₄ Fettalkohol mit 7 bis 11 Ethoxylateinheiten, beispielsweise Laureth-9.

Bevorzugte nichtionische Tenside sind auch Alkylfettsäureamide, Fettalkoholalkoxylate (alkoxy = ethoxy und/oder propoxy) und Fettalkoholpolyglucoside, wobei die Alkylkette des Fettalkohols bzw. der Fettsäure ein gesättigter oder ungesättigter, linearer oder verzweigter Alkylrest mit 8 bis 22 Kohlenstoffatomen sein kann. Beispiele geeigneter nichtionische Tenside sind z. B. Laureth-7, Laureth-9, Ceteareth-25 (Sympatens Marken, Kolb AG), Coco Glucoside, Decyl Glucoside Lauryl Glucoside. Verwendet werden die INCI Bezeichnungen, INCI = International Nomenclature of Cosmetic Ingredients.

Bei den amphoteren Tensiden sind besonders bevorzugt Alkylbetaine und Alkylamidopropylbetaine wie Laurylbetain, Myristylbetain (Chembetaine BW, Lubrizol), Cocamidopropylbetain, Undecylenamidopropylbetain (Rewoteric AM BU 185, Evonik) Capryl/Capramidopropylbetain (z. B. Tego Betain 810, Evonik) oder aber Imidazolinderivate wie Cocoamphoacetate (Rewoteric AMC, Evonik) und Cocoamphodiacetate (Rewoteric AM2CNM, Evonik) sowie Alkylhydroxysultaine (Chembetaine CAS, Chembetaine LHS, Lubrizol). Speziell bevorzugt sind amphotere Tenside mit Betain-Struktur.

In einer bevorzugten Ausgestaltung der erfindungsgemäßen Mittel liegen die Tenside insbesondere in einer Menge von 0,2 bis 0,6 Gew.-%, jeweils bezogen auf das Gesamtgewicht des Mittels, vor.

Weiterhin können die erfindungsgemäßen Mittel als weitere Bestandteile Salze, vorzugsweise in einer Menge von 0,001 bis 2,0 Gew.-%, enthalten, so beispielsweise Natriumchlorid, Kaliumchlorid, Calciumchlorid, Natriumcarbonat, Natriumhydrogencarbonat und deren Mischungen. Der Einsatz von Salzen kann beispielsweise für die Einstellung der isotonischen Osmolalität des resultierenden Mittels verwendet werden.

Bei Bedarf können die Mittel weiterhin Pflegestoffe aufweisen, z. B. mehrwertige Alkohole wie Popan-1,2-diol, Butan-1,3-diol, Pentan-1,2-diol, Hexan-1,2-diol, Octan-1,2-diol, Dekan-1,2-diol, Propan-1,2,3-triol, Sorbitol, aber auch andere Pflegestoffe wie Dexpanthenol, Allantoin und Urea. Die Pflegestoffe können beispielsweise in einer Menge von 0,001 bis 10 Gew.-% enthalten sein.

Als geeignete Alkandiole haben sich insbesondere solche herausgestellt, die 3 bis 12 Kohlenstoffatome aufweisen. Besonders bevorzugt sind Alkandiole ausgewählt aus der Gruppe bestehend aus 1,2 Propylenglykol, 1,3-Butylenglykol, 1,2-Pentandiol, 1,2-Hexandiol, 1,6-Hexandiol und/oder 1,2-Octandiol. Bevorzugt sind 1,2-Alkandiole mit 5 bis 10 Kohlenstoffatomen, insbesondere 1,2 Octandiol.

Die Alkandiole werden bevorzugt in Mengen von 0,01 bis 10 Gew.-%, insbesondere 0,05 bis 5 Gew.-%, eingesetzt.

Weiterhin kann die Hilfsstoffmatrix einen oder mehrere Komplexbildner in einer Menge von 0,001 bis 5,0 % enthalten. Geeignete Komplexbildner sind z. B. Ethylendiamintetraacetat, Gluconsäure, Gluconodeltalacton, Methylglycindiessigsäure Natriumsalz, Tetranatrium-N,N-bis(carboxylatomethyl)-L-glutamat und Polyasparaginsäure.

Weiterhin können die Mittel ein oder mehrere basische Mittel zur Einstellung des pH Wertes in einen Bereich zwischen 4 und 9 in vorzugsweise in einer Menge von 0,0001 bis 1,0 Gew.-% enthalten. Geeignete Mittel sind beispielsweise Natriumhydroxid, Kaliumhydroxid, Ammoniumhydroxid, Alkanolamine, insbesondere Monoethanolamin, Triethanolamin, Tetrahydroxypropylethylendiamin (Neutrol TE, BASF), Tris(hydroxymethyl)-aminomethan (bekannt unter der Bezeichnung TRIS), und 2-Amino-2-Methyl-1 Propanol (Dow).

Bei Bedarf können die Mittel ein oder mehrere saure Mittel zur Einstellung des pH Wertes in einen Bereich zwischen 4 und 9 in vorzugsweise in einer Menge von 0,0001 bis 1,0 % enthalten. Geeignete Mittel sind beispielsweise Salzsäure, Phosphorsäure, Schwefelsäure, Essigsäure, Citronensäure, Äpfelsäure, Milchsäure und/oder Glykolsäure.

Weiterhin können die erfindungsgemäßen Mittel Stoffe zur Einstellung der Viskosität vorzugsweise in einer Menge von 0,001 bis 10,0 Gew.-% enthalten. Geeignete Viskositätsregler sind beispielsweise nichtionische Polymere auf Cellulosebasis Methylcellulose, Ethylcellulose, Hydroxyethylcellulose (z. B. Natrosol Typen Ashland), Hydroxypropylmethylcellulose (z. B. Methocel Typen, Dow), Hydroxypropylmethylcellulose (Klucel Typen, Hercules) sowie synthethische Polymere wie Polyacrylamid und Polyvinylpyrrolidon.

Weiterhin können die Mittel einen oder mehrere Polyalkylenglykol(e) enthalten, insbesondere wenn eine viskose oder salbenartige Konsistenz erwünscht ist.

Die mittleren relativen Molmassen der Polyalkylenglykole liegen vorzugsweise im Bereich von 200 bis 10000, insbesondere von 400 bis 6000. Bei den nachfolgend beschriebenen verschiedenen Polyalkylenglykolen können bestimmte Bereiche nochmals besonders vorteilhaft sein.

Erfindungsgemäß bevorzugt einzusetzen sind lineare oder verzweigte, insbesondere lineare Polyalkylenglykole der allgemeinen Formel HO-[R-O]n-H, in der R für (CH2)2, CH2CH(CH3), CH2CH(CH2CH3), und/oder für (CH2)4 und n für Werte bzw. Mittelwerte von 2 bis etwa 200, vorzugsweise 3 bis 190, weiter bevorzugt 4 bis 180, besonders bevorzugt 6 bis 150, insbesondere 10 bis 120 stehen. Die Polyalkylenglykole können durch ringöffnende Polymerisation von Ethylenoxid, Propylenoxid und/oder Tetrahydrofuran hergestellt werden. Es sind dies im einzelnen die Polyethylenglykole mit R = (CH2)2, die Polypropylenglykole mit R = CH2CH(CH3), die Polytetrahydrofurane mit R = (CH2)4 und die Copolymere aus Ethylenoxid, Propylenoxid und/oder Tetrahydrofuran.

Erfindungsgemäß bevorzugt einzusetzen sind Polyethylenglykole (PEG) mit einer mittleren relativen Molmasse von 200 bis 10000, insbesondere von 400 bis 6000. Für Polyethylenglykole existieren verschiedene Nomenklaturen, die zu Verwirrungen führen können. Technisch gebräuchlich ist die Angabe des mittleren relativen Molgewichts im Anschluss an die Angabe "PEG", so dass "PEG 200" ein Polyethylenglykol mit einer relativen Molmasse von ca. 190 bis ca. 210 charakterisiert. Gemäß INCI-Nomenklatur wird das Kurzzeichen PEG mit einem Bindestrich versehen und folgt direkt auf den Bindestrich eine Zahl, die der Zahl n in der obigen allgemeinen Formel entspricht. Kommerziell erhältliche Polyethylenglykole sind beispielsweise PEG 200/PEG-4, PEG 300/PEG-6, PEG-7, PEG-8, PEG 400, PEG-9, PEG-10, PEG-12, PEG 600, PEG-14, PEG-16, PEG 800/PEG-18, PEG-20, PEG 1000, PEG 1200, PEG 1500/PEG-32, PEG-40, PEG 2000, PEG-55, PEG-60, PEG 3000, PEG 3350/PEG-75 und PEG 4000/PEG-90, wobei die Bezeichnungen gemäß den beiden Nomenklaturen für einander entsprechende Polyethylenglykole durch das Zeichen "/" getrennt nebeneinander gestellt sind. Die kommerziell erhältlichen Polyethylenglykole sind beispielsweise unter den Handelsnamen Carbowax® (Union Carbide), Emkapol® und Renex® PEG (ICI), Lipoxol® (DEA), Polyglykol® E (Dow), Pluracol® E, Pluriol® E sowie Lutrol® E (BASF) verfügbar.

Polypropylenglykole (PPG) sind über einen breiten Molmassenbereich von 250 (PPG-4) bis 4.000 (PPG-69) klare, nahezu farblose Flüssigkeiten, für deren Bezeichnung die zuvor beschriebene INCI-Nomenklatur analog verwendet wird. So werden die Polypropylenglykole der obigen allgemeinen Formel mit Werten n von 5 bzw. 6 als PEG-5 bzw. PEG-6 bezeichnet. Die niedermolekularen Polypropylenglykole sind mit Wasser mischbar, während die höhermolekularen Vertreter weniger wasserlöslich sind. Kommerziell erhältlich sind beispielsweise die gemäß INCI bezeichneten Polypropylenglykole PPG-7, PPG-9, PPG-12, PPG-13, PPG-15, PPG-17, PPG-20, PPG-26, PPG-30, PPG-33, PPG-34, PPG-51 und PPG-69. Bezugsquellen sind dem International Cosmetic Ingredient Dictionary and Handbook zu entnehmen.

Bei den Copolymeren handelt es sich vorzugsweise um statistische Copolymere und insbesondere um Blockcopolymere aus Ethylen- und Propylenoxid, Ethylenoxid und Tetrahydrofuran, Propylenoxid und Tetrahydrofuran oder Ethylenoxid, Propylenoxid und Tetrahydrofuran, bevorzugt aus Ethylen- und Propylenoxid, besonders bevorzugt um Blockcopolymere aus Ethylen- und Propylenoxid.

Erfindungsgemäß bevorzugt einzusetzen sind statistische Copolymere aus a Ethylen- und b Propylenoxideinheiten sind beispielsweise folgende gemäß International Cosmetic Ingredient Dictionary and Handbook als PEG/PPG-a/b bezeichnete Copolymere (Molmasse), wobei a und b Mittelwerte darstellen: PEG/PPG-18/4 Copolymer (1000), PEG/PPG-17/6 Copolymer (1100), PEG/PPG-35/9 Copolymer (2100) und PEG/PPG-23/50 Copolymer (3900).

Bevorzugte Blockcopolymere aus Ethylen- und Propylenoxid genügen der Formel HO(CH2CH2O)x(CH(CH3)CH2O)y(CH2CH2O)x'H in der x und x' für Mittelwerte von 2 bis 130 und y für Mittelwerte von 15 bis 67 stehen, und werden mit dem internationalen Freinamen Poloxamer bezeichnet, der auch im International Cosmetic Ingredient Dictionary and Handbook verwendet wird. Jedes Poloxamer ist durch eine dreistellige Nummer gekennzeichnet. Die ersten beiden Ziffern geben multipliziert mit 100 die durchschnittliche Molmasse des Polypropylenglykol-Anteils und die letzte Ziffer multipliziert mit 10 den Polyethylenglykol-Anteil in Gew.-% an. Dieser beträgt 10 bis 80 Gew.-%, vorzugsweise nicht mehr als 50 Gew.-%, insbesondere nicht mehr als 40 Gew.-%, besonders bevorzugt nicht mehr als 30 Gew.-%, beispielsweise 10, 20 oder 30 Gew.-%. Die Herstellung der Poloxamere erfolgt zweistufig, wobei zunächst Propylenoxid kontrolliert an Propylenglykol addiert und der erhaltene Polypropylenglykolblock durch anschließende Addition von Ethylenoxid von zwei Polyethylenglykol-Blöcken eingefasst wird. Besonders bevorzugte Blockcopolymere sind beispielsweise folgende flüssige Poloxamer-Typen (x, y, x'; Molmasse; z. T. Schmelzpunkt): Poloxamer 101 (2, 16, 2; 1100; -32), Poloxamer 122 (5, 21, 5; 1630; -26), Poloxamer 123 (7, 21, 7; 1900; -1), Poloxamer 105 (11, 16, 11; 1850; 7), Poloxamer 181 (3, 30, 3; 2000; -29), Poloxamer 124 (11, 21, 11; 2200; 16), Poloxamer 182 (8, 30, 8; 2500; -4), Poloxamer 183 (10, 30, 10; 2650; 10), Poloxamer 212 (8, 35, 8; 2750; -7), Poloxamer 231 (6, 39, 6; 2750; -37), Poloxamer 184 (13, 30, 13; 2900; 16), Poloxamer 185 (19, 30, 19; 3400), Poloxamer 282 (10, 47, 10; 3650; 7), Poloxamer 331 (7, 54, 7; 3800; -23), Poloxamer 234 (22, 39, 22; 4200; 18), Poloxamer 401 (6, 67, 6; 4400; 5), Poloxamer 284 (21, 47, 21; 4600) und Poloxamer 402 (13, 67, 13; 5000; 20). Kommerziell sind die Poloxamere unter den Handelsnamen Pluronic® und Synperonic® PE erhältlich, den ein Buchstabe aus der Gruppe L, P und F sowie eine zwei- oder dreistellige Zahl nachgestellt ist. Dabei ist die letzte Ziffer mit der letzten Ziffer der Poloxamer-Nomenklatur identisch und ergeben die davorstehenden ein- oder zweistelligen Zahlen multipliziert mit 300 die ungefähre Molmasse des Polypropylenglykol-Anteils bzw. mit 3 multipliziert in etwa die aus den ersten beiden Ziffern der Poloxamer-Nomenklatur-Nummer gebildeten Zahl, d. h. entsprechen 3, 4, 6, 7, 8, 9, 10 und 12 in dieser Reihenfolge den zweistelligen Zahlen 10, 12, 18, 21, 23, 28, 33 und 40 am Anfang der Nummer gemäß der Poloxamer-Nomenklatur. Die Buchstaben unterscheiden flüssige (L), pastöse (P) und feste (F) Poloxamere. So ist beispielsweise das Poloxamer 101 als Pluronic® L 31 und Synperonic® PE L 31 erhältlich.

Eine weitere Klasse geeigneter Blockcopolymere aus Ethylen- und Propylenoxid genügen der Formel HO(CH(CH3)CH2O)y(CH2CH2O)x(CH2CH(CH3)O)y'H. Hier wird ein Polyethylenglykol-Block von zwei Polypropylenglykol-Blöcken eingerahmt, während bei den Poloxameren ein Polypropylenglykol-Block von zwei Polyethylenglykol-Blöcken eingefasst wird. Die Herstellung erfolgt wiederum zweistufig, wobei zunächst Ethylenoxid kontrolliert an Ethylenglykol addiert und der erhaltene Polyethylenglykol-Block durch anschließende Addition von Propylenoxid von zwei Polypropylenglykol-Blöcken eingefasst wird. Kommerziell erhältlich sind diese Blockcopolymere wie die Poloxamere unter dem Handelsnamen Pluronic®(BASF), dem jeweils ein alphanumerischer Code aus drei Ziffern und dem zwischen die zweite und dritte Ziffer geschobenen Buchstaben R folgt. Die Bedeutung der Ziffern ist mit der Bedeutung im Rahmen der Poloxamer-Nomenklatur identisch. Der eingeschobene Buchstabe R (für engl. reverse) deutet auf die im Vergleich zu den Poloxameren invertierte Struktur hin. Bevorzugte Vertreter dieser Klasse sind die folgenden Pluronic®-Typen (Molmasse; Schmelzpunkt): Pluronic®10R5 (1950; 15), Pluronic®12R3 (1800; -20), Pluronic® 17R1 (1900; -27), Pluronic®17R2 (2150; -25), Pluronic®17R4 (2650; 18), Pluronic®25R1 (2700; -5), Pluronic®25R2 (3100; - 5), Pluronic®31R1 (3250; -25) und Pluronic®31R2 (3300; 9).

Weiterhin können die erfindungsgemäßen Mittel geeignete lipophile Substanzen enthalten, ausgewählt aus Vaseline, Paraffinöl, verzweigten Paraffinen wie Isohexadekan oder C13-14 Isoparaffin, Fettsäureestern wie Isopropylmyristat, Isopropylpalmitat, Octylstearat, 2-Ethylhexylstearat sowie Triglyceride ungesättiger und/oder gesättigter Fettsäuren wie beispielsweis mittelkettige Triglyceride, Sojaöl, Sonnenblumenöl, Rhizinusöl, hydriertes Rhizinusöl und Triisostearin.

Weiterhin können die erfindungsgemäßen zusätzlich ein organisches Isotonisierungsmittel, beispielsweise Glycerin enthalten. Das Isotonisierungsmittel liegt üblicherweise in einer Menge von bis zu 15 Gew.-%, vorzugsweise 0,5 bis 10 Gew.-%, weiter bevorzugt 2 bis 9 Gew.-%, vor.

Weiterhin können die Mittel zusätzlich Antioxidantien aufweisen. Dies ist insbesondere von Bedeutung wenn die Mittel als Emulsionen formuliert sind. Hier können die Antioxidantien zur Stabilisierung der Formulierung beitragen. Bevorzugt eingesetzt wird beispielsweise α-Tocopherol. Üblicherweise werden die Antioxidantien in einer Menge von bis zu 0,3 Gew.-%, vorzugsweise 0,05 bis 0,2 Gew.-%, eingesetzt.

Zur Wirkungsverstärkung können die erfindungsgemäßen Mittel weiterhin zusätzlich eine lipophile Substanz mit synergistischer antimikrobieller Wirkung enthalten. Hierzu eignen sich insbesondere ätherische Öle.

Das Mittel, welches zur Behandlung und Prophylaxe von Infektionen durch Bakterien verwendet wird, umfasst die folgenden Bestandteile:
a) 0,05 bis 4 Gew.-% eines kationischen Oligomers oder Polymers bestehend aus einer chemisch modifizierten Hydroxyalkylcellulose, bevorzugt Polyquaternium-4 und/oder Polyquaternium-10,
b) 0,001 und 2 Gew.-% Polylysin und/oder Polyarginin, speziell epsilon-Polylysin, beispielsweise epsilon-Poly-L-lysin,
c) 0,1 bis 0,8 Gew.-% nichtionische und/oder amphotere Tenside und
d) mindestens 85 Gew.-%, vorzugsweise mindestens 90 Gew.-%, speziell mindestens 95 Gew.-% Wasser und/oder Polyalkylenglykole, wobei sich die Gewichtsangaben jeweils auf das Gesamtgewicht des Mittels beziehen.

Zur Herstellung der erfindungsgemäßen Mittel können diese in dem Fachmann geläufigen Schritten vermischt und vorzugsweise sterilisiert werden.

Ein weiterer Aspekt der Offenbarung ist ein Verfahren zur Reduzierung multiresistenter Bakterien, insbesondere Betalaktam-Antibiotika und Glycopeptid-Antibiotika resistenter Bakterien, auf einer unbelebten oder belebten Oberfläche, umfassend die folgenden Schritte:
i) Applizieren des erfindungsgemäßen Mittels auf die Oberfläche und
ii) Einwirkenlassen des Mittels, wobei in einer Ausführungsform Behandlungsmethoden des menschlichen oder tierischen Körpers ausgeschlossen sind.

Das Verfahren kann zur Reduzierung oder auch Entfernung von multiresistenten Bakterien auf unbelebten Oberflächen, wie beispielsweise harten Oberflächen, wie etwa Krankenhauseinrichtungsgegenständen, chirurgischen Gegenständen, Gegenstände, die mit multiresistenten Bakterien kontaminierten Patienten in Kontakt gekommen sind, eingesetzt werden. Bevorzugt wird es auf unbelebten Oberflächen, wie etwa der Haut, Wunden oder Schleimhäuten, die mit multiresistenten Bakterien kontaminiert sind, eingesetzt. Das Applizieren der Mittel kann mit Applikatoren, vorzugsweise einem mit dem Mittel getränkten Tuch, erfolgen.

Zur Anwendung können die Produkte auf die Haut aufgetragen werden, ggf. mit Unterstützung eines indifferenten Schwammes, eines Tupfers oder eines Tuchs aus Polyester oder Polypropylen. Das Tuch kann mit der Lösung vorgetränkt sein, was die Anwendung vereinfacht. Nach der Anwendung des Produktes kann die Haut entweder mit sauberem Wasser abgewaschen werden, um einen physikalischen Reinigungseffekt zu erzielen. Bevorzugt kann das Produkt zur Zeitersparnis nach dem Abtrocknen auch auf der Haut abtrocknen und somit verbleiben.

Weiterhin können die Mittel in der Nase appliziert werden, bei Bedarf kann ein Gel oder eine Salbe auf einen geeigneten indifferenten Tupfer oder Wattestäbchen aufgetragen und der so vorbereitete Gegenstand in das Nasenloch eingeführt werden. Alternativ kann das Gel oder die Salbe auch mittels einer geeigneten Tube und Applikator in die Nase eingebracht werden.

Ein weiterer Gegenstand der Erfindung ist ein Kit umfassend ein erfindungsgemäßes Mittel in einem Behälter und räumlich getrennt davon einen Applikator zum Auftragen des Mittels.

Ein weiterer Gegenstand der Erfindung ist ein Applikator umfassend eine Aufnahmevorrichtung und ein erfindungsgemäßes Mittel.

Die erfindungsgemäßen Mittel können bevorzugt als Antiseptikum, insbesondere für Wunden und Körperhöhlenspülungen eingesetzt werden. Es ist daher von Vorteil, den pH-Wert der erfindungsgemäßen Mittel in einen Bereich von 5 bis 9, vorzugsweise 5,5 bis 7 und insbesondere zwischen 6 und 7 einzustellen.

Ein weiterer Aspekt der Offenbarung ist eine pharmazeutische Zubereitung, die die erfindungsgemäße Mittel umfasst bzw. aus ihr besteht.

Ein weiterer Aspekt der Offenbarung ist ein Antiseptikum, welches die erfindungsgemäßen Mittel umfasst.

In einem Aspekt der Offenbarung wird das Antiseptikum zur Prophylaxe oder Behandlung der Haut, der Schleimhaut, des Genitalbereichs, des Auges, sowie von Wunden eingesetzt. Ein bevorzugter Gegenstand ist daher das Antiseptikum zur Verwendung in der Prophylaxe oder Behandlung von Infektionen der Haut, der Schleimhaut, des Genitalbereichs, des Auges, sowie von Wunden.

Insbesondere geeignet sind die Antiseptika für Körperhöhlenspülungen, Gelenkspülungen, als Augenantiseptikum, als Bestandteil von Wundauflagen, als Wundantiseptikum, zur Nasenhöhlenantiseptik, insbesondere bei MRSA-Besiedlung, zur Genital- und Mundhöhlenantiseptik sowie zur Hautantiseptik.

Die pharmazeutische Zubereitung ist besonders geeignet zur Prophylaxe und Therapie von Infektionen, insbesondere von Infektionen an empfindlichen Körperregionen, wie beispielsweise dem Peritoneum, der Bauchhöhle, der Harnblase, des Harnleiters, des zentralen Nervensystems und hyalinem Gelenkknorpel.

Darüber hinaus werden die pharmazeutischen Zubereitungen zur Prophylaxe oder Therapie atopischer Dermatiden, infizierter Ekzeme und von Dermatomykosen eingesetzt. Ein bevorzugter Gegenstand ist daher die pharmazeutische Zubereitung zur Verwendung in der Prophylaxe oder Therapie atopischer Dermatiden, infizierter Ekzeme und von Dermatomykosen.

Ein weiterer Gegenstand der Offenbarung ist die Verwendung der Emulsion zur Wundbehandlung, beispielsweise zur Herstellung einer Wundauflage, die insbesondere fest oder gelartig ist. In einem Aspekt wird hierzu das Trägermaterial der Wundauflage mit dem Mittel berieselt, besprüht oder imprägniert oder in eine gelartige Wundauflagen eingearbeitet.

Die Erfindung wird durch die nachfolgenden Beispiele näher beschrieben, ohne jedoch darauf beschränkt zu sein.

### Beispiele

Um das Wesen der Erfindung zu demonstrieren, wurden Rezepturen nachfolgender Zusammensetzungen hergestellt. Sofern nicht anders erwähnt, handelt es sich bei den Angaben in Prozent um Massenprozent (Gew.-%), also um "Gramm pro 100 Gramm".

### Vergleichsbeispiel 1

Hierbei handelt es sich um eine wässrig gelartige Zusammensetzung enthaltend eine Hydroxyethylcellulose, die nicht kationisch modifiziert ist (z. B. Natrosol 250 MR, Ashland).

Die Lösung wurde durch Vermischen der Bestandteile bei Raumtemperatur hergestellt.

| Rezepturbestandteil | Menge in Gew.-% |
|---|---|
| Hydroxyethylcellulose | 1,8 % |
| Glycerin | 8,6 % |
| Laureth-9 | 0,40 % |
| Octan-1,2-diol | 0,20 % |
| Gereinigtes Wasser | 89,0 % |

### Beispiel 2

Die Lösung wurde durch Vermischen der Bestandteile bei Raumtemperatur hergestellt.

| Rezepturbestandteil | Menge in Gew.-% |
|---|---|
| Polyquaternium-4 | 0,50 % |
| Laureth-9 | 0,40 % |
| Polylysin ¹⁾ | 0,01 % |
| Octan-1,2-diol | 0,20 % |
| Gereinigtes Wasser | 98,89 % |

| | |
|---|---|
| ¹⁾ epsilon-Poly-L-lysin; Mw : 5000 g/mol | |

Die Mittel gemäss Beispiel 1 und 2 sowie die Einzelkomponenten wurden in einem Reihenverdünnungstest auf minimale Hemmkonzentrationen (MIC nach 24 h) gegenüber Methicillin resistentem Staphylococcus aureus (Krankenhausisolat) untersucht. Im Fall der Einzelkomponenten wurden als Maximalkonzentrationen die in Vergleichsbeispiel 1 angegebenen Werte, verdünnt in sterilem, gereinigtem Wasser als Ausgangswerte genommen. Die Verdünnungen betrugen 1:2, 1:4, 1:8 und 1:16. Die einzelnen Proben wurden in Müller Hinton II Bouillon (kationen-adjustiert, Lieferant z. B. Becton Dickinson) kultiviert, welche zur Empfindlichkeitsprüfung schnell wachsender grampositiver und gramnegativer Bakterien gegenüber Antibiotika verwendet wird. In Vergleichsbeispiel 1 liess sich in den Verdünnungen in keinem Fall eine nennenswerte Hemmung der Bakterienkulturen zeigen.

Demgegenüber zeigte das Mittel in Beispiel 2 eine signifikante Hemmung der Bakterienkulturen nach 24 Stunden bereits bei einer Verdünnung von 1:8.

### Vergleichsbeispiel 3

Die Rezeptur in Beispiel 3 ist eine wässrige Lösung von Polyquaternium-4 (Celquat L-200) mit Polyarginin in gereinigtem Wasser. Die Lösung wurde durch Vermischen der Bestandteile bei Raumtemperatur hergestellt.

| Rezepturbestandteil | Menge in Gew.-% |
|---|---|
| Polyquaternium-4 | 0,5 % |
| Polyarginin | 0,5 % |
| gereinigtes Wasser | 99,0 % |

### Vergleichsbeispiel 4

Hierbei handelt es sich um eine Formulierung mit einer Mischung von Polyquaternium-4, Polyquaternium-10 als auch Polylysin, die als Lösung vorliegt.

| Rezepturbestandteil | Menge in Gew.-% |
|---|---|
| Polyquaternium-10 | 0,25 % |
| Polyquaternium-4 | 0,25 % |
| Polylysin ¹⁾ | 0,5 % |
| Gereinigtes Wasser | 99,0 % |

| | |
|---|---|
| ¹⁾ epsilon-Poly-L-lysin; Mw : 5000 g/mol | |

### Vergleichsbeispiel 5

Bei diesem Beispiel handelt es sich um eine einer Mischung von Polyquaternium-4 und Polylysin in einer isotonischen wässrigen Umgebung, die zur Spülung von kolonisierten Wunden angewendet werden kann. Die Lösung lässt sich durch Vermischen der Komponenten herstellen. Der pH-Wert wird zweckmässig mit Salzsäure bzw. Natriumhydroxidlösung auf einen Wert von 7 eingestellt. Die homogene Lösung kann in ein geeignetes Behältnis aus Glas, Polyethylen oder Polypropylen eingefüllt und verschlossen werden. Zur Gewährleistung der Keimfreiheit kann die Lösung unter aseptischen Bedingungen hergestellt und im Blow Fill Seal Verfahren abgefüllt werden. Die Konzentrationsangaben in diesem Beispiel sind aufgeführt in g/V Prozent, also in Gramm pro 100 Milliliter.

| Rezepturbestandteil | Menge in g/V % |
|---|---|
| Polyquaternium-4 | 0,20 % |
| Polylysin ¹⁾ | 0,10 % |
| Natriumchlorid | 0,86 % |
| Kaliumchlorid | 0,30 % |
| Calziumchlorid x2H₂O | 0,33 % |
| Gereinigtes Wasser | ad 100 % |

| | |
|---|---|
| ¹⁾ epsilon-Poly-L-lysin; Mw : 5000 g/mol | |

### Beispiel 6

Bei dieser Mischung von Polyquaternium-4 in einer isotonischen wässrigen Umgebung, die zur Spülung von Wunden angewendet werden kann, wird ein amphoteres Tensid zugesetzt, welches die Reinigung unterstützen kann. Der pH-Wert wird zweckmässig mit Salzsäure bzw. Natriumhydroxidlösung auf einen Wert von 7 eingestellt. Zur Gewährleistung der Keimfreiheit kann die Lösung auf Temperaturen oberhalb von 100 °C erwärmt und somit sterilisiert werden. Die Konzentrationsangaben in diesem Beispiel sind aufgeführt in g/V Prozent, also in Gramm pro 100 Milliliter.

| Rezepturbestandteil | Menge in g/V % |
|---|---|
| Polyquaternium-4 | 0,20 % |
| Polylysin ¹⁾ | 0,01 % |
| Capryl/Capramidopropylbetain | 0,10 % |
| Natriumchlorid | 0,86 % |
| Kaliumchlorid | 0,30 % |
| Calziumchlorid x2H₂O | 0,33 % |
| Gereinigtes Wasser | ad 100 % |

| | |
|---|---|
| ' epsilon-Poly-L-lysin; Mw : 5000 g/mol | |

### Vergleichsbeispiel 7

Es handelt sich um eine Formulierung mit einer Mischung von Polyquaternium-4 und Polyhexamethylenbiguanid in einer isotonisch wässrigen Umgebung. Auch hier wird der pH-Wert mit Salzsäure bzw. Natriumhydroxidlösung auf einen Wert von 7 eingestellt. Die resultierende Konzentration an Polyhexamethylenbiguanid beträgt 0,02 %. Die Konzentrationsangaben in diesem Beispiel sind aufgeführt in g/V Prozent, also in Gramm pro 100 Milliliter.

| Rezepturbestandteil | Menge in g/V % |
|---|---|
| Polyquaternium-4 | 0,20 % |
| Polyhexamethylenbiguanid 20 % | 0,10 % |
| Natriumchlorid | 0,86 % |
| Kaliumchlorid | 0,30 % |
| Calziumchlorid x2H₂O | 0,33 % |
| Gereinigtes Wasser | ad 100 % |

Das Wirkungsspektrum sowie Wirkungsleistung dieser Zusammensetzung ist jedoch begrenzt.

### Beispiel 8

Bei Beispiel 8 handelt es sich um eine Ausführung als Lösung, die zum Waschen Abreiben oder Pflegen auf die Haut aufgetragen werden kann.

| Rezepturbestandteil | Menge in Gew.-% |
|---|---|
| Polyquaternium-4 | 0,50 % |
| Laureth-9 | 0,40 % |
| Polylysin ¹⁾ | 0,20 % |
| Octan-1,2-diol | 0,20 % |
| Allantoin | 0,10 % |
| Gereinigtes Wasser | ad 100 % |

| | |
|---|---|
| ¹⁾ epsilon-Poly-L-lysin; Mw : 5000 g/mol | |

### Beispiel 9

Bei Beispiel 9 handelt es sich um eine Ausführung als Lösung, die zum Waschen Abreiben oder Pflegen auf die Haut aufgetragen werden kann.

| Rezepturbestandteil | Menge in Gew.-% |
|---|---|
| Polyquaternium-4 | 0,50 % |
| Laureth-9 | 0,40 % |
| Polylysin ¹⁾ | 0,20 % |
| Allantoin | 0,10 % |
| Gereinigtes Wasser | ad 100 % |

| | |
|---|---|
| ¹⁾ epsilon-Poly-L-lysin; Mw : 5000 g/mol | |

### Vergleichsbeispiel 10

Bei Beispiel 10 handelt es sich um die Ausführung als Gel, welches zur Reinigung und Behandlung der Nasenschleimhaut angewendet werden kann.

| Rezepturbestandteil | Menge in Gew.-% |
|---|---|
| Polyquaternium-4 | 0,5 % |
| Polylysin ¹⁾ | 0,1 % |
| Hydroxyethylcellulose | 1,8 % |
| Glycerin | 8,6 % |
| Gereinigtes Wasser | ad 100 % |

| | |
|---|---|
| ¹⁾ epsilon-Poly-L-lysin; Mw : 5000 g/mol | |

### Vergleichsbeispiel 11

Bei Beispiel 11 handelt es sich um die Ausführung als Gel, welches eine Abwandlung von Beispiel 10 mit einer höheren Viskosität darstellt.

| Rezepturbestandteil | Menge in Gew.-% |
|---|---|
| Polyquaternium-4 | 1,0 % |
| Polylysin ¹⁾ | 0,2 % |
| Hydroxyethylcellulose | 3,0 % |
| Glycerin | 8,6 % |
| TRIS | 0,05 % |
| Gereinigtes Wasser | 87.35 % |

| | |
|---|---|
| ¹⁾ epsilon-Poly-L-lysin; Mw : 5000 g/mol | |

### Vergleichsbeispiel 12

Bei Beispiel 12 handelt es sich um die Ausführung als hydrophile Salbe zur Behandlung der Nasenschleimhaut. Die Salbe kann in einer Emulgiermaschine vom Typ Fryma Koruma VME12 hergestellt werden.

| Rezepturbestandteil | Menge in Gew.-% |
|---|---|
| Polyquaternium-4 | 0,50 % |
| Polylysin ¹⁾ | 0,01 % |
| Polyethylenglykol 400 | 55 % |
| Polyethylenglykol 4000 | 35 % |
| Tetrahydroxypropylethylendiamin | 0,05 % |
| Gereinigtes Wasser | 9,44 % |

| | |
|---|---|
| ¹⁾ epsilon-Poly-L-lysin; Mw : 5000 g/mol | |

In weiteren Reihenverdünnungstests zeigten ausgewählte Rezepturen auch eine Hemmwirkung gegen Pseudomonas aeruginosa und Enterococcus faecium. Desweiteren konnte in vitro eine deutliche Reduktion von Methicillin resistentem Staphylococcus aureus innerhalb von einer bis 3 Stunden nachgewiesen werden. Auch Pseudomonas aeruginosa wurde wirksam reduziert. In einer unter Praxisbedingungen durchgeführten klinischen Untersuchung konnte nach einem Dekolonisationszyklus eine erfolgreiche Dekolonisation bei 30 % MRSA positiver Patienten gezeigt werden, so dass die Patienten am 28. Tag MRSA frei waren. Dies entspricht der Dekolonisationsrate, die im Journal Antimicrob Agents Chemother. 1999 Jun;43(6): 1412-6 publiziert wurde. Dabei wurden weder ein Antibiotikum wie Mupirozin noch ein Biozid wie Chlorhexidin verwendet. In dermatologischen Prüfungen wiesen ausgewählte Rezepturen eine sehr gute Haut- und Schleimhautverträglichkeit auf. Auffallend war auch die ausgezeichnete Gewebeverträglichkeit bei Wunden. Die gute Verträglichkeit konnte im praktischen Einsatz bestätigt werden.

### Beispiel 13 und Vergleichsbeispiel 14

### Vergleich Polyquaternium 4 und Polylysin versus Polyquaternium 4 und Polihexanid (PHMB)

### Die Ergebnisse stammen aus Hemmhoftests in Anlehnung an die DIN 58940

Bei den folgenden Rezepturen wurden gleiche Stoffmengen Polihexanid und Polylysin eingesetzt. Als Tensid wurde Capryl/ Capramidopropyl Betain des Herstellers Evonik eingesetzt, welches durch eine besonders gute Schleimhaut- und Gewebeverträglichkeit bekannt ist.

Der Hemmhoftest wurde in Anlehnung an die DIN 58940:2007 als Agarlochtest durchgeführt. Hierzu wurden Agarplatten hergestellt. Die Agarplatten enthielten als Bakterienkulturen jeweils entweder Staphylococcus aureus ATCC 6538 oder Pseudomonas aeruginosa ATCC 15442. In die Agaroberfläche wurde jeweils ein kreisrundes Loch gestanzt, in dieses Loch wurde die entsprechende Produktprüflösung appliziert. Nach 24 Stunden Inkubationszeit wurden die wachstumsfreien Zonen, also der optisch sichtbare Hemmhof rund um das Loch, in welches die Produktprüflösung appliziert worden war, vermessen. Die ermittelten Hemmhöfe (Angabe in mm) demonstrieren eine mikrobizide bzw. mikrobistatische Aktivität der Produktprüflösung. Wird kein Hemmhof festgestellt, kann davon ausgegangen werden, das die Lösung gegenüber dem Test-Bakterienkultur nicht mikrobizid oder mikrobistatisch aktiv ist.

| Rezepturbestandteil | Beispiel 13 Menge in Gew.-% | | Vergleichsbeispiel 14 Menge in Gew.-% | |
|---|---|---|---|---|
| Polyquaternium-4 | 0,50 | | 0,50 | |
| Polylysin ¹⁾ (Molmasse 5000) | 0,20 | | -- | |
| Polihexanid (Molmasse ca. 2500) | -- | | 0,10 | |
| Tego Betain 810 (Capryl/ Capramidopropyl Betain) | 0,35 | | 0,35 | |
| Gereinigtes Wasser | ad 100 | | ad 100 | |
| Hemmhof S. aureus | 2 | 2 | 5 | 5 |
| Hemmhof P. aeruginosa | 3 | 2 | 0 | 0 |

| | | | | |
|---|---|---|---|---|
| ¹⁾ epsilon-Poly-L-lysin; Mw : 5000 g/mol | | | | |

Die Ergebnisse zeigen bei der Kombination aus Polyquaternium-4 und Polylysin eine ausgeglichene Wirkung gegenüber grampositiven (S. aureus) und gramnegativen (P. aeruginosa) Keimen. Demgegenüber zeigte die Kombination aus Polyquaternium-4 und Polihexanid zwar eine Wirksamkeit gegen grampositive Keime, nicht jedoch gegen gramnegative Keime wie Pseudomonas.

Die Hemmhoftests zeigten für die Einzelsubstanzen 0,50 % Polyquaternium-4 und Polyquaternium-10 sowie Polylysin in Wasser keine Aktivität. Die Aktivität konnte durch Zusatz mindestens eines Tensids gesteigert werden, was durch Beispiel 15 gezeigt wird. Die Tenside weisen allein jedoch keine antimikrobielle Aktivität auf.

### Beispiel 15

Eine wirksame erfindungsgemässe Rezeptur mit einem nichtionischen Tensid demonstriert Beispiel 15.

| Rezepturbestandteil | Beispiel 15 Menge in Gew.-% | |
|---|---|---|
| Polyquaternium-4 | 0,50 | |
| Polylysin ¹⁾ | 0,20 | |
| Laureth 9 | 0,40 | |
| Allantoin | 0,10 | |
| Gereinigtes Wasser | ad 100 | |
| Hemmhof S. aureus | 9 | 10 |
| Hemmhof P. aeruginosa | 2.5 | 2 |

| | | |
|---|---|---|
| ¹⁾ epsilon-Poly-L-lysin; Mw : 5000 g/mol | | |

Die Aufgabe, antibiotikafreie und gut verträgliche Produkte zu entwickeln, mit der sich mit multiresistenten grampositiven und/oder gramnegativen Keimen kolonisierte Haut, Schleimhaut und Wunden dekolonisieren sowie sich Infektionen behandeln oder vermeiden lassen, wurde damit zufriedenstellend gelöst.

## Patentansprüche

1. Mittel zur Verwendung in der therapeutischen Behandlung oder Prophylaxe von Infektionen durch Betalaktam-Antibiotika oder Glycopeptid-Antibiotika resistente Bakterien, umfassend
a) 0,05 bis 4 Gew.-% eines kationischen Oligomers oder Polymers, bestehend aus einer chemisch modifizierten Hydroxyalkylcellulose, und
b) 0,001 bis 2 Gew.-% Polylysin und/oder Polyarginin,
c) 0,1 bis 0,8 Gew.-% nicht-ionisches und/oder amphoteres Tensid, und
d) mindestens 85 Gew.-% Wasser und/oder Polyalkylglykole,
wobei die Gewichtsangaben jeweils bezogen sind auf das Gesamtgewicht des Mittels.

2. Mittel zur Verwendung gemäß Anspruch 1, **dadurch gekennzeichnet, dass** die Bakterien Methicillin- und/oder Oxacillin- und/oder Carbapenem und/oder Vancomycin-resistent sind.

3. Mittel zur Verwendung gemäß Anspruch 1 oder 2, **dadurch gekennzeichnet, dass** das Mittel topisch, insbesondere auf Haut oder Schleimhaut oder Wunden appliziert wird.

4. Mittel zur Verwendung gemäß einem oder mehreren der Ansprüche 1 bis 3, **dadurch gekennzeichnet, dass** das kationische Oligomer oder Polymer in einer Menge von 0,1 bis 3 Gew.-%, speziell von 0,15 bis 2 Gew.-%, insbesondere von 0,25 bis 1 Gew.-%, jeweils bezogen auf das Gesamtgewicht des Mittels, vorliegt.

5. Mittel zur Verwendung gemäß einem oder mehreren der Ansprüche 1 bis 4, **dadurch gekennzeichnet, dass** das kationische Oligomer oder Polymer eine chemisch modifizierte Hydroxyethylcellulose ist und insbesondere das kationische Oligomer oder Polymer quaternäre Ammoniumgruppen aufweist.

6. Mittel zur Verwendung gemäß einem oder mehreren der Ansprüche 1 bis 5, **dadurch gekennzeichnet, dass** das kationische Oligomer oder Polymer eine Ladungsdichte von wenigstens 0,1 meq/g, bevorzugt wenigstens 1,0 meq/g, weiter bevorzugt wenigstens 2.0 meq/g, besonders bevorzugt wenigstens 3,0 meq/g, speziell bevorzugt 4,0 meq/g und insbesondere wenigstens 5,0 meq/g oder wenigstens 6,0 meq/g, bestimmt gemäß Kolloidtitration, aufweist.

7. Mittel zur Verwendung gemäß einem oder mehreren der Ansprüche 1 bis 6, **dadurch gekennzeichnet, dass** das kationische Oligomer oder Polymer ausgewählt ist aus der Gruppe bestehend aus Polyquaternium-4, Polyquaternium-4/Hydroxypropyl Stärke Copolymer, Polyquaternium-10 und Polyquaternium-24.

8. Mittel zur Verwendung gemäß einem oder mehreren der Ansprüche 1 bis 7, **dadurch gekennzeichnet, dass** die biozide Komponente b) epsilon-Polylysin, beispielsweise epsilon-Poly-L-lysin, ist.

9. Mittel zur Verwendung gemäß einem oder mehreren der Ansprüche 1 bis 8, **dadurch gekennzeichnet, dass** die Komponente b) eine Polyaminosäure ist, die 15 bis 50, vorzugsweise 20 bis 40 und speziell 25 bis 35 Aminosäure-Monomereinheiten, Lysin oder Arginin, aufweist.

10. Mittel zur Verwendung gemäß einem oder mehreren der Ansprüche 1 bis 9, **dadurch gekennzeichnet, dass** es Wasser in einer Menge oberhalb von 87 Gew.-%, besonders bevorzugt oberhalb von 89 Gew.-%, insbesondere oberhalb von 95 Gew.-% und speziell oberhalb von 98 Gew.-% oder oberhalb 99 Gew.-%, enthält, wobei die Gewichtsangaben bezogen sind auf das Gesamtgewicht des Mittels.

11. Mittel zur Verwendung gemäß einem oder mehreren der Ansprüche 1 bis 10, **dadurch gekennzeichnet, dass** das Mittel in Form einer Lösung, einer Emulsion, eines Gels, einer Salbe oder Creme vorliegt.

12. Mittel zur Verwendung gemäß einem oder mehreren der Ansprüche 1 bis 11, **dadurch gekennzeichnet, dass** das nichtionische Tensid, vorzugsweise Fettalkoholethoxylate, insbesondere C₈₋₂₂-Fettalkohole mit 5 bis 30 Ethoxylateinheiten, speziell C₁₀₋₁₄ Fettalkohol mit 7 bis 11 Ethoxylateinheiten, beispielsweise Laureth-9 umfasst und insbesondere das amphotere Tensid eine Betain-Struktur aufweist und wobei die Tenside bevorzugt in einer Menge von 0,2 bis 0,6 Gew.-%, jeweils bezogen auf das Gesamtgewicht des Mittels, vorliegen.

13. Mittel gemäß einem oder mehreren der Ansprüche 1 bis 12 zur therapeutischen Verwendung in der Reduzierung Betalaktam-Antibiotika und Glycopeptid-Antibiotika resistenter Bakterien, auf einer belebten Oberfläche, umfassend die folgenden Schritte:
i) Applizieren eines Mittels umfassend
a) 0,05 bis 4 Gew.-% eines kationischen Oligomers oder Polymers, bestehend aus einer chemisch modifizierten Hydroxyalkylcellulose, und
b) 0,001 bis 2 Gew.-% Polylysin und/oder Polyarginin,
c) 0,1 bis 0,8 Gew.-% nicht-ionisches und/oder amphoteres Tensid, und
d) mindestens 85 Gew.-% Wasser und/oder Polyalkylglykole, wobei die Gewichtsangaben jeweils bezogen sind auf das Gesamtgewicht des Mittels, auf die Oberfläche und
ii) Einwirkenlassen des Mittels.

14. Kit umfassend ein Mittel gemäß einem oder mehreren der Ansprüche 1 bis 12 in einem Behälter und räumlich getrennt davon einen Applikator zum Auftragen des Mittels.

15. Applikator umfassend eine Aufnahmevorrichtung und ein Mittel gemäß einem oder mehreren der Ansprüche 1 bis 12.

## Claims

1. An agent for use in therapeutic treatment or prophylaxis of infections by bacteria resistant to beta-lactam antibiotics or glycopeptide antibiotics, comprising
a) from 0.05 to 4% by weight of a cationic oligomer or polymer consisting of a chemically modified hydroxyalkylcellulose, and
b) from 0.001 to 2% by weight polylysine and/or polyarginine;
c) from 0.1 to 0.8 % by weight non-ionic and/or amphoteric surfactant, and
d) at least 85% by weight water and/or polyalkylglycols,
wherein the weight percentages are respectively based on the total weight of the agent.

2. The agent for use according to claim 1, **characterized in that** said bacteria are resistant to methicillin and/or oxacillin and/or carbapenem and/or vancomycin.

3. The agent for use according to claim 1 or 2, **characterized in that** said agent is applied topically, especially on the skin or mucosa or wounds.

4. The agent for use according to one or more of claims 1 to 3, **characterized in that** said cationic oligomer or polymer is present in an amount of from 0.1 to 3% by weight, especially from 0.15 to 2% by weight, particularly from 0.25 to 1% by weight, respectively based on the total weight of the agent.

5. The agent for use according to one or more of claims 1 to 4, **characterized in that** said cationic oligomer or polymer is a chemically modified hydroxyethylcellulose.

6. The agent for use according to one or more of claims 1 to 5, **characterized in that** said cationic oligomer or polymer has a charge density of at least 0.1 meq/g, preferably at least 1.0 meq/g, more preferably at least 2.0 meq/g, more preferably at least 3.0 meq/g, especially preferably at least 4.0 meq/g, and in particular, at least 5.0 meq/g, or at least 6.0 meq/g, as determined by colloid titration.

7. The agent for use according to one or more of claims 1 to 6, **characterized in that** said cationic oligomer or polymer is selected from the group consisting of polyquaternium-4, polyquaternium-4/hydroxypropylstarch copolymer, polyquaternium-10, and polyquaternium-24.

8. The agent for use according to one or more of claims 1 to 7, **characterized in that** said biocidal component b) is epsilon-polylysine, for example, epsilon-poly-L-lysine.

9. The agent for use according to one or more of claims 1 to 8, **characterized in that** said component b) is a poly(amino acid) having from 15 to 50, preferably from 20 to 40, and especially from 25 to 35, lysine or arginine amino acid monomer units.

10. The agent for use according to one or more of claims 1 to 9, **characterized by** containing water in an amount above 87% by weight, more preferably above 89% by weight, especially above 95% by weight, and particularly above 98% by weight, or above 99% by weight, wherein the weight percentages are based on the total weight of the agent.

11. The agent for use according to one or more of claims 1 to 10, **characterized in that** said agent is in the form of a solution, an emulsion, a gel, an ointment, or a cream.

12. The agent for use according to one or more of claims 1 to 11, **characterized in that** said non-ionic surfactant preferably comprises fatty alcohol ethoxylates, especially C₈₋₂₂ fatty alcohols with 5 to 30 ethoxylate units, especially C₁₀₋₁₄ fatty alcohol with 7 to 11 ethoxylate units, for example, laureth-9, and in particular, said amphoteric surfactant has a betain structure, wherein said surfactants are preferably present in an amount of 0.2 to 0.6% by weight, respectively based on the total weight of the agent.

13. The agent according to one or more of claims 1 to 12 for therapeutic use in the reduction of bacteria resistant to beta-lactam antibiotics and glycopeptide antibiotics, on an animate surface, comprising the following steps:
i) applying an agent comprising
a) from 0.05 to 4% by weight of a cationic oligomer or polymer consisting of a chemically modified hydroxyalkylcellulose, and
b) from 0.001 to 2% by weight polylysine and/or polyarginine;
c) from 0.1 to 0.8 % by weight non-ionic and/or amphoteric surfactant, and
d) at least 85% by weight water and/or polyalkylglycols, wherein the weight percentages are respectively based on the total weight of the agent,
to the surface, and
ii) allowing the agent to act.

14. A kit comprising an agent according to one or more of claims 1 to 12 in a container, and spatially separated therefrom, an applicator for applying the agent.

15. An applicator comprising a receiving device and an agent according to one or more of claims 1 to 12.

## Revendications

1. Agent à utiliser dans le traitement ou la prophylaxie thérapeutique d'infections par des bactéries résistantes aux antibiotiques bêta-lactames ou aux antibiotiques glycopeptide, comprenant
a) 0,05 à 4 % en poids d'un oligomère ou polymère cationique consistant en hydroxyalkylcellulose chimiquement modifiée, et
b) 0,001 à 2 % en poids de polylysine et/ou polyarginine,
c) 0,1 à 0,8 % en poids d'un tensioactif non ionique et/ou amphotère, et
d) au moins 85 % en poids d'eau et/ou polyalkylglycole,
les pourcentages en poids étant respectivement relatifs au poids total dudit agent.

2. Agent à utiliser selon la revendication 1, **caractérisé en ce que** lesdites bactéries sont résistantes à la méticilline et/ou à l'oxacilline et/ou au carbapénème et/ou à la vancomycine.

3. Agent à utiliser selon la revendication 1 ou 2, **caractérisé en ce que** ledit agent est appliqué par voie topique, notamment sur la peau ou la muqueuse ou sur des blessures.

4. Agent à utiliser selon l'une ou plusieurs des revendications 1 à 3, **caractérisé en ce que** ledit oligomère ou polymère cationique est présent en une quantité de 0,1 à 3 % en poids, spécifiquement de 0,15 à 2 % en poids, notamment de 0,25 à 1 % en poids, respectivement par rapport au poids total dudit agent.

5. Agent à utiliser selon l'une ou plusieurs des revendications 1 à 4, **caractérisé en ce que** ledit oligomère ou polymère cationique est une hydroxyéthylcellulose chimiquement modifiée, notamment ledit oligomère ou polymère cationique présente des groupes ammonium quaternaires.

6. Agent à utiliser selon l'une ou plusieurs des revendications 1 à 5, **caractérisé en ce que** ledit oligomère ou polymère cationique présente une densité de charge d'au moins 0,1 meq/g, de préférence au moins 1,0 meq/g, de préférence encore au moins 2,0 meq/g, de préférence encore au moins 3,0 meq/g, de préférence encore au moins 4,0 meq/g, et notamment au moins 5,0 meq/g ou au moins 6,0 meq/g, telle que déterminée par titration colloïdale.

7. Agent à utiliser selon l'une ou plusieurs des revendications 1 à 6, **caractérisé en ce que** ledit oligomère ou polymère cationique est choisi dans le groupe consistant en polyquaternium-4, copolymère de polyquaternium-4/hydroxypropylamidon, polyquaternium-10, et polyquaternium-24.

8. Agent à utiliser selon l'une ou plusieurs des revendications 1 à 7, **caractérisé en ce que** le composant biocide b) est l'epsilon-polylysine, par exemple, l'epsilon-poly-L-lysine.

9. Agent à utiliser selon l'une ou plusieurs des revendications 1 à 8, **caractérisé en ce que** le composant b) est un poly(acide aminé) qui possède 15 à 50, de préférence 20 à 40, et notamment 25 à 35 unités monomères d'acides aminés de lysine ou arginine.

10. Agent à utiliser selon l'une ou plusieurs des revendications 1 à 9, **caractérisé en ce qu'**il contient de l'eau en une quantité supérieure à 87 % en poids, de préférence supérieure à 89 % en poids, notamment supérieure à 95 % en poids, et spécifiquement supérieure à 98 % en poids, ou supérieure à 99 % en poids, les pourcentages en poids étant respectivement relatifs au poids total dudit agent.

11. Agent à utiliser selon l'une ou plusieurs des revendications 1 à 10, **caractérisé en ce que** ledit agent est sous la forme d'une solution, d'une émulsion, d'un gel, d'une pommade, ou d'une crème.

12. Agent à utiliser selon l'une ou plusieurs des revendications 1 à 11, **caractérisé en ce que** ledit tensioactif non ionique de préférence comprend des éthoxylates d'alcool gras, notamment des alcool gras en C₈₋₂₂ avec 5 à 30 unités d'éthoxylate, spécifiquement alcool gras en C₁₀₋₁₄ avec 7 à 11 unités d'éthoxylate, par exemple, laureth-9, et notamment ledit tensioactif amphotère présente une structure de bétaïne, et dans lequel les tensioactifs sont de préférence présents en une quantité de 0,2 à 0,6% en poids, respectivement relatifs au poids total dudit agent.

13. Agent selon l'une ou plusieurs des revendications 1 à 12 pour l'utilisation thérapeutique dans la réduction de bactéries résistantes aux antibiotiques bêta-lactames et aux antibiotiques glycopeptide sur une surface animée, comprenant les étapes consistant à :
i) appliquer un agent comprenant
a) 0,05 à 4 % en poids d'un oligomère ou polymère cationique consistant en hydroxyalkylcellulose chimiquement modifiée, et
b) 0,001 à 2 % en poids de Polylysine et/ou polyarginine,
c) 0,1 à 0,8 % en poids d'un tensioactif non ionique et/ou amphotère, et
d) au moins 85 % en poids d'eau et/ou polyalkylglycole, les pourcentages en poids étant respectivement relatifs au poids total dudit agent,
sur la surface et
ii) laisser la surface sous l'influence dudit agent.

14. Trousse comprenant un agent selon l'une ou plusieurs des revendications 1 à 12 dans un récipient et séparément un applicateur pour appliquer ledit agent.

15. Applicateur comprenant un dispositif de réception et un agent selon l'une ou plusieurs des revendications 1 à 12.
